# EUROPEAN PATENT APPLICATION

(11) **EP 2 246 359 A1**
(43) Date of publication of application: **03.11.2010**
(21) Application number: 09382055.3
(22) Date of filing: 23.04.2009
(51) Int. Cl.: C07J 7/00, C07J 51/00, C07J 71/00

(54) **Process for obtaining fluorometholone and intermediates therefor**

(71) Applicant: Crystal Pharma, S.L.U., 47151 Boecillo - Valladolid (ES)
(72) Inventor: LORENTE BONDE-LARSEN, Antonio, E-47151, Boecillo - Valladolid (ES); IGLESIAS RETUERTO, Jesús Miguel, E-47151, Boecillo - Valladolid (ES); HERRÁIZ SIERRA, Ignacio, E-47151, Boecillo - Valladolid (ES); BERMEJO GONZÁLEZ, Francisco, E-37008, Salamanca (ES); MARCOS ESCRIBANO, José Andrés, E-37008, Salamanca (ES); GUTIÉRREZ FUENTES, Luis Gerardo, E-47151, Boecillo - Valladolid (ES)
(74) Representative: ABG Patentes, S.L.

(57) **Abstract**

The present invention relates to a process for obtaining compounds of formula (I) and (V), intermediates useful in the synthesis of some steroids, for example, fluorometholone and derivatives thereof. The invention also relates to other intermediates useful in synthesis.

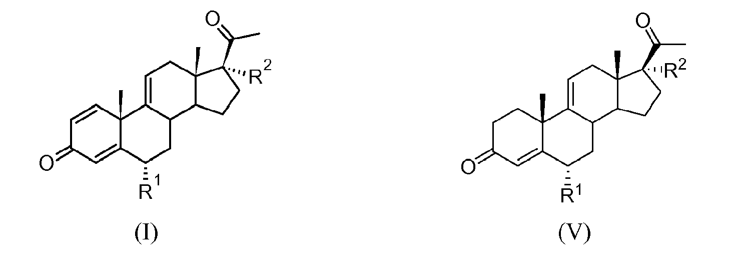

## Description

### FIELD OF THE INVENTION

The present invention relates to a process for obtaining derivatives of steroids or solvates thereof, as well as to intermediate\s useful in said process. More particularly, it relates to a process for preparing derivatives useful for obtaining fluorometholone and derivatives thereof, such as fluorometholone acetate.

### BACKGROUND OF THE INVENTION

The active ingredient fluorometholone and its corresponding acetate in position 17, referred to as fluorometholone acetate, are corticosteroids with anti-inflammatory properties useful in dermatology and ophthalmology since they do not affect intraocular pressure. The chemical structure of these active ingredients is shown in Scheme 1 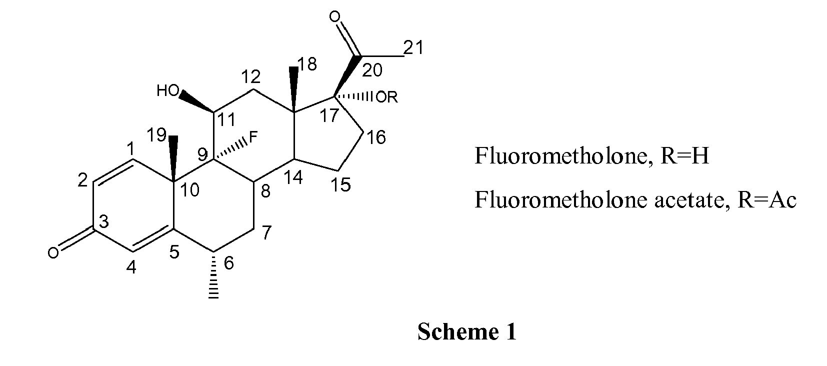 in which the numbers that are usually assigned to the carbons of the molecules of said family is also indicated, which numbers are followed in this description.

Fluorometholone was first described in US 2,867,637**,** a document in which several processes for obtaining it are also described.

A first process allows obtaining fluorometholone by means of the elimination, in three synthetic steps, of the 21-hydroxyl group of the compound 9α-fluoro-11β,17,21-trihydroxy-6α-methyl-1,4-pregnadiene-3,20-dione **(1).** Compound **1** is a very advanced starting product, obtained in turn in 5 synthetic steps from methylprednisolone 21-acetate (J. Am. Chem. Soc. 1957, 79, 1515). Therefore, the formation of fluorometholone involves a total of 8 synthetic steps starting from a commercial product, as shown in Scheme 2.

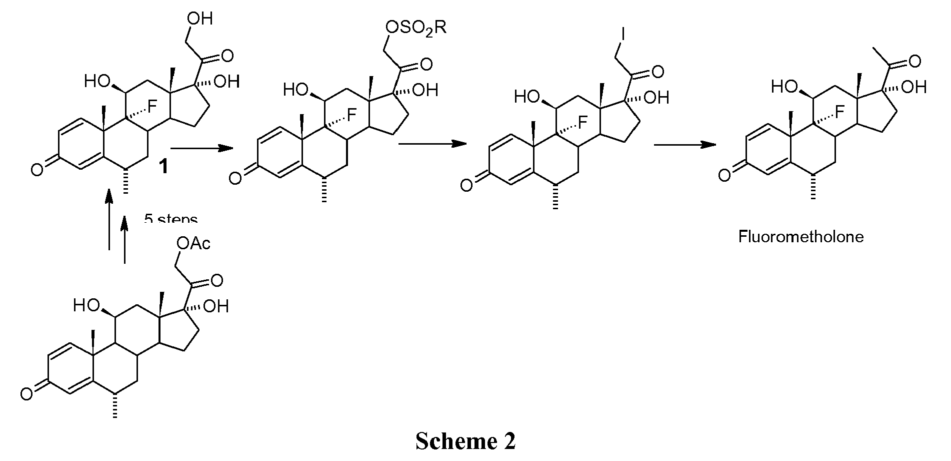

A second process disclosed in Example 6 of US 2,867,637 starts from 11β,17-dihydroxy-6α-methyl-1,4-pregnadiene-3,20-dione **(2),** a compound which is not commercially available and for which the synthesis thereof is not described. Therefore, this fluorometholone synthesis process, in addition to the transformations necessary for obtaining compound **2,** requires 5 synthetic steps to convert said compound into fluorometholone, as shown in Scheme 3.

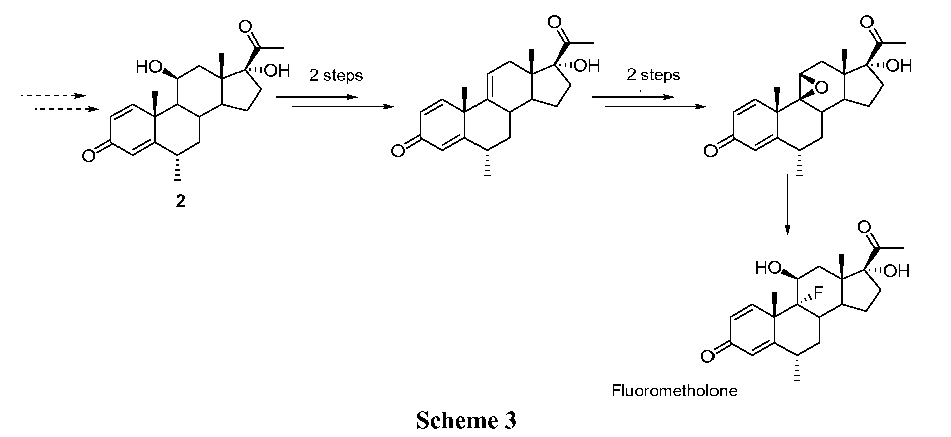

Patent US 2,867,638 uses 11β,17-dihydroxy-6α-methyl-4-pregnene-3,20-dione **(3)** as a starting product, performing in the last step a dehydrogenation in position 1-2 by fermentation, making difficult the industrial application of the process. A large number of steps are also required in this case, since the starting product **3** is not easily available either (see Scheme 4).

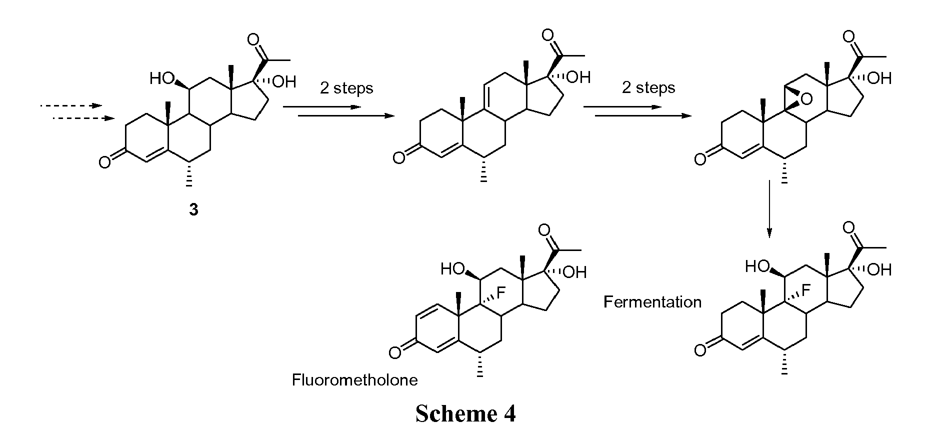

FR 1 330 110 uses 17-α-hydroxyprogesterone, as a starting product, but 12 synthetic steps are needed to obtain fluorometholone. Therefore, although the starting product is commercially available, the synthesis is long (see Scheme 5), and furthermore 11β-hydroxylation and 1,2-dehydrogenation are carried out by fermentation, which limits its industrial application.

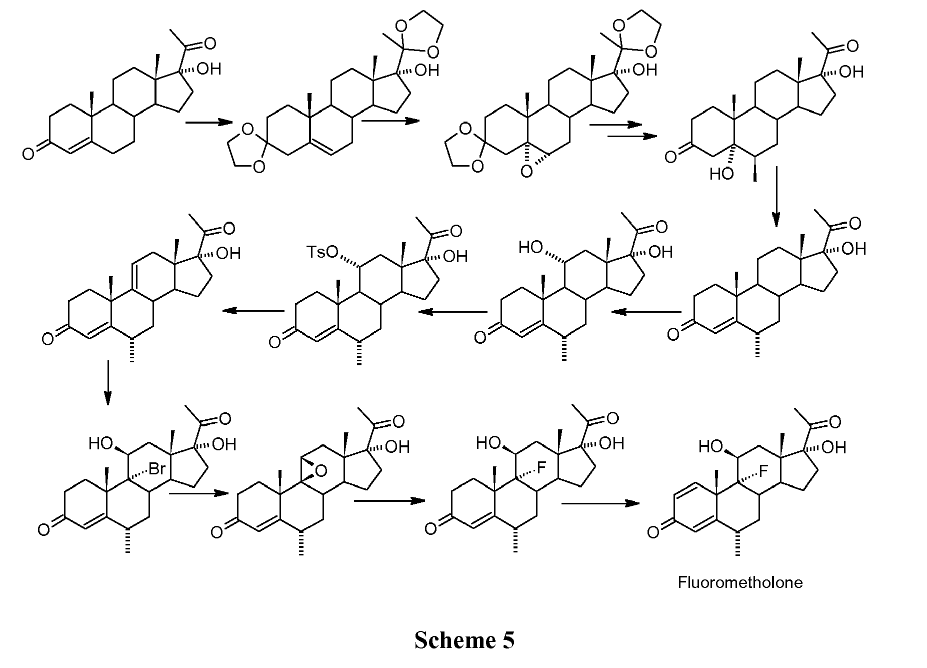

Another alternative for obtaining fluorometholone is provided in FR 2 223 364**.** In this case the starting product is 9α-fluoro-11β,17-dihydroxy-1,4-pregnadiene-3,20-dione **(4),** on which the methyl group is introduced in position 6 and it is finally dehydrogenated in position 1-2 by fermentation or using 2,3-dicyano-5,6-dichloroparabenzoquinone (DDQ) in dioxane under reflux (see Scheme 6).

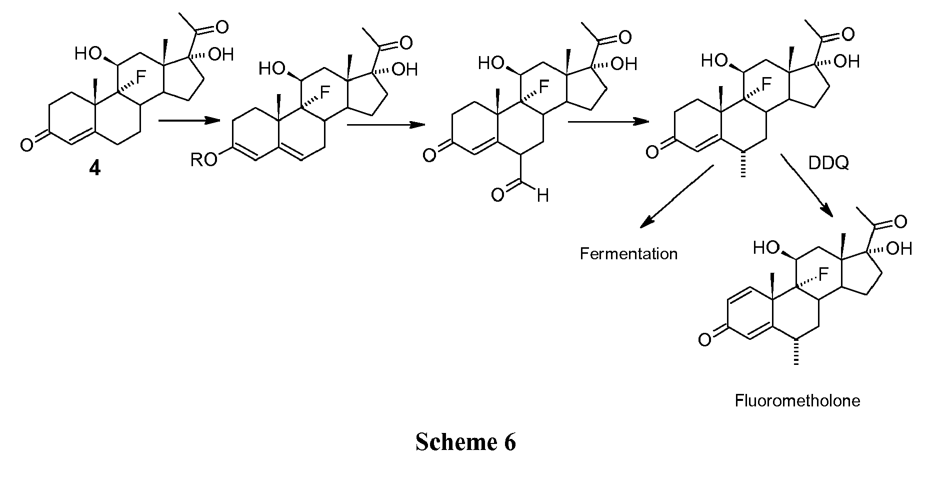

However, the main difficulty of the final dehydrogenation with DDQ of the previous process is the possible low regioselectivity in the reaction, positions 1-2 and 5-6 being able to compete. Furthermore, under these conditions, the recrystallization mentioned in the patent is not sufficient, and the crude reaction product obtained requires purification by means of a chromatography column. As an additional problem, 9α-fluoro-11β,17-dihydroxy-4-pregnene-3,20-dione is a non-commercially available starting product. Its formation is necessary, and therefore the number of steps for obtaining fluorometholone from a commercial product increases.

In relation to fluorometholone acetate, the acetylation of fluorometholone is described in J. Org. Chem. 1960, 25, 1675. More recently, patent US 4,346,037 describes base-catalyzed acylations using enol esters, such as isopropenyl acetate, on substrates of a steroid nature.

It is therefore necessary to develop an alternative process for obtaining fluorometholone and derivatives thereof (for example, fluorometholone acetate) overcoming all or part of the problems associated to known processes. In particular, said alternative process must start from a commercial product, must take place in few synthetic steps and prevent fermentation processes which make its industrialization difficult.

### SUMMARY OF THE INVENTION

The present invention provides an efficient process for obtaining derivatives of 6α-alkyl-4,9(11)-diene-pregna-3,20-dione of general formula (V) or solvates thereof, forming synthetic intermediates useful in the preparation of steroids, particularly of fluorometholone and derivatives thereof, such as fluorometholone acetate.

According to a first aspect, the present invention is aimed at a process for obtaining a compound of formula (V) or a solvate thereof, 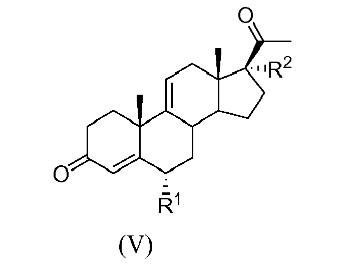 wherein
R¹ is C₁-C₆ alkyl;
R² is -OR³, -OC(=O)R⁴ or -O-(HPG), wherein
R³ is H, C₁-C₆ alkyl or C₆-C₁₄ aryl;
R⁴ is H or C₁-C₆ alkyl; and
HPG is a hydroxyl protecting group;
comprising the catalytic hydrogenation of a compound of formula (III) or a solvate thereof, 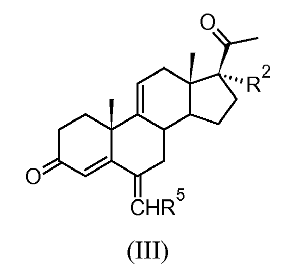 wherein R² is as defined above, and R⁵ is hydrogen or C₁-C₅ alkyl.

An additional aspect of the invention relates to said compounds of formula (III) or solvates thereof, intermediates useful in the synthesis of steroids, for example, in the synthesis of fluorometholone or the acetate thereof in position 17.

An additional aspect relates to a process for preparing a compound of formula (III) or a solvate thereof, comprising subjecting a compound of formula (II) or a solvate thereof to a vinylogous Mannich reaction, 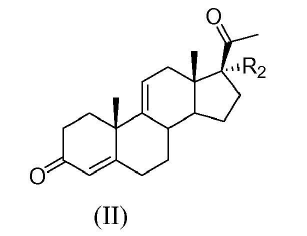 wherein R² is as defined above.

The compounds of formula (V) or solvates thereof can be transformed by dehydrogenation in a surprisingly efficient manner into the corresponding 6α-alkyl-1,4,9(11)-triene-pregna-3,20-dione derivatives of general formula (I) or solvates thereof. The dehydrogenation of the compounds of formula (V) comprises the regioselective formation of a silyl enol ether and subsequent oxidation in the presence of a quinone and a base B. Therefore, an additional aspect of the invention is a process for obtaining a compound of formula (I) or a solvate thereof, 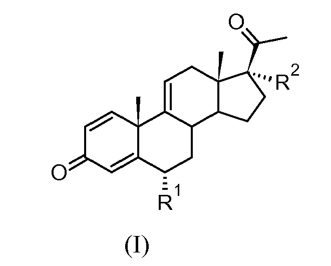 wherein
R¹ is C₁-C₆ alkyl;
R² is OR³, OC(=O)R⁴ or O-(HPG), wherein
R³ is H, C₁-C₆ alkyl or C₆-C₁₄ aryl;
R⁴ is H or C₁-C₆ alkyl; and
HPG is a hydroxyl protecting group;
comprising
forming a silyl enol ether derivative of a compound of formula (V) or a solvate thereof, 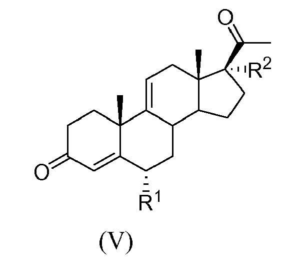 wherein R¹ and R² are as defined above;
and then reacting said resulting silyl enol ether with a quinone and a base B.

When R² is hydroxyl in the starting compound of formula V, said process can provide protected derivatives of formula (Ia) or solvates thereof, 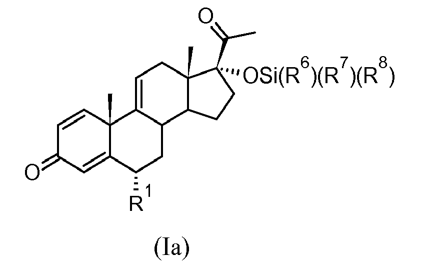 wherein R¹ is as defined above and wherein R⁶, R⁷ and R⁸, independently of one another, represent C₁-C₆ alkyl or C₃-C₆ cycloalkyl.

An additional aspect of the invention relates to said compounds of formula (Ia) or solvates thereof, intermediates useful in the synthesis of steroids, for example, in the synthesis of fluorometholone or the acetate thereof in position 17.

The compounds of formula (Ia) can be obtained as essentially the only product of the reaction or as mixtures together with other compound or compounds of formula (I).

Therefore, an additional aspect of the invention is a process for synthesizing a compound of formula (Ia) or a solvate thereof, comprising forming a silyl enol ether derivative of a compound of formula (V) or a solvate thereof, 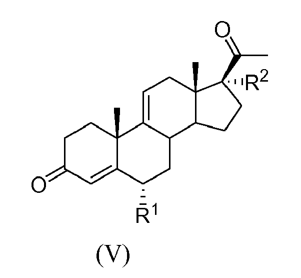 wherein R¹ is as defined above, and R² is hydroxyl;
and then reacting said resulting silyl enol ether with a quinone and a base B.

The catalytic hydrogenation of the compounds of formula (III) to obtain compounds of formula (V) can occur through compounds of formula (IV) 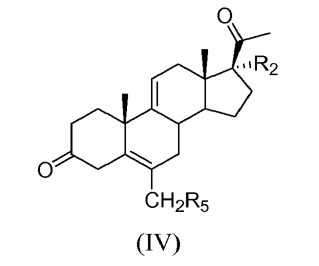 wherein R⁵ and R² are as defined above.

When these compounds of formula (IV) are formed, they are directly transformed into compounds of formula (V) or they can be isolated and then transformed into compounds of formula (V) in a subsequent step.

Therefore, an additional aspect relates to a process for obtaining compounds of formula (V) or solvates thereof, comprising reacting a compound of formula (IV) or a solvate thereof in an acid medium.

An additional aspect of the invention relates to said compounds of formula (IV) or solvates thereof, intermediates useful in the synthesis of steroids, for example, in the synthesis of fluorometholone or the acetate thereof in position 17.

An additional aspect relates to a process for preparing a compound of formula (IV) or a solvate thereof, comprising the catalytic hydrogenation, preferably in the presence of a base A, of a compound of formula (III) or a solvate thereof, 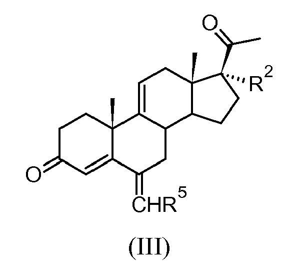 wherein R⁵ and R² are as defined above.

The present invention has a number of advantages which contribute to provide a process suitable for its industrial application. Firstly, it is possible to obtain fluorometholone and derivatives thereof from commercial products in few synthetic steps. The conditions described in the present invention allow obtaining the different intermediates with high yields and enable their isolation with a high purity without having to use expensive purification techniques (for example, chromatography). The process of the invention furthermore only involves chemical reactions, which prevents using fermentation processes, facilitating its industrial application.

The hydrogenation is regioselective in the presence of the double bond of position 9-11 of the compound of formula (III), which has the consequence of substantially reducing the synthesis steps necessary for introducing the remaining functionalities of fluorometholone and derivatives thereof.

Additional aspects of the present invention are also the use of the compounds of formula (Ia), (III) and (IV) or solvates thereof in the preparation of fluorometholone and derivatives thereof, such as fluorometholone acetate.

### DETAILED DESCRIPTION OF THE INVENTION

### Definitions

As used herein, the term "C₁-C₆ alkyl" relates to a radical derived from a linear or branched alkane, with 1 to 6 carbon atoms, for example, methyl, ethyl, propyl, butyl, etc., optionally substituted with one or more substituents independently selected from halogen, C₆-C₁₄ aryl and C₁-C₆ alkyl. An example of substituted alkyl is benzyl.

As used herein, the term "C₃-C₆ cycloalkyl" relates to a radical derived from a cycloalkane, with 3 to 6 carbon atoms, for example, cyclopropyl, cyclobutyl, etc., optionally substituted with one or more substituents independently selected from halogen, aryl and C₁-C₆alkyl.

As used herein, the term "C₆-C₁₄ aryl" relates to a radical derived from an aromatic hydrocarbon, with 6 to 14 carbon atoms, for example, phenyl, tolyl, xylyl, etc., optionally substituted with one or more substituents independently selected from halogen and C₁-C₆alkyl.

As used herein, the term "C₁-C₆ alkoxyl" relates to an O-alkyl radical, with 1 to 6 carbon atoms, for example, methoxy, ethoxy, propoxy, butoxy, etc., optionally substituted with one or more substituents independently selected from halogen and alkyl C₁-C₆.

As used herein, the term "halogen" or "halo" relates to fluorine, chlorine, bromine or iodine.

As used herein, the term "hydroxyl protecting group" (HPG) includes any group capable of protecting a hydroxyl group. Illustrative examples of hydroxyl protecting groups have been described by Green TW et al. in "Protective Groups in Organic Synthesis", 3rd Edition (1999), Ed. John Wiley & Sons (ISBN 0-471-16019-9). Virtually any hydroxyl protecting group can be used to put the invention into practice; nevertheless, in a particular embodiment, the hydroxyl protecting group is an ester group or an ether group, which can be converted into a hydroxyl group under mild conditions. Illustrative, non-limiting examples include C₁-C₆ alkyl (for example, methyl, ethyl, methyl ether, t-butyl, benzyl, etc.), or silyl, for example, a silyl radical of general formula Si(R⁶)(R⁷)(R⁸), wherein R⁶, R⁷ and R⁸, independently of one another, represent C₁-C₆ alkyl or C₃-C₆ cycloalkyl. Representative examples of silyl groups are trimethylsilyl or dimethyl-*t*-butylsilyl.

Likewise, the compounds used in the process described by the present invention can be obtained in free form or in solvate form. In both cases, they are preferably obtained in crystalline form, both as free compounds or as solvates (for example, hydrates), both forms being included within the scope of the present invention. Solvation methods are generally well known in the state of the art.

### Preparation of the compounds of formula (V)

The catalytic hydrogenation of the compounds of formula (III) is performed using conditions known in the state of the art. See, for example, pages 1002-1007 of March, J. "Advanced Organic Chemistry: Reactions, Mechanisms and Structure", Wiley-Interscience, fifth edition; or pages 193-198 of Carey, F. and Sundberg, J. R. "Advanced Organic Chemistry - Part B: Reactions and Synthesis" Plenum, second edition. Preferably, molecular hydrogen used as a hydrogen source and Pd/C as a catalyst.

According to a particular embodiment, the catalytic hydrogenation is performed in the presence of a base A. The presence of base A improves the selectivity of the reduction even more. According to a particular embodiment, said base A is an organic or inorganic base, preferably a trialkylamine, particularly, a trialkylamine of formula N(R₁₀)₃, wherein each R₁₀ is independently selected from a C₁-C₆ alkyl. According to a particular embodiment, said trialkylamine is selected from the group consisting of trimethylamine, triethylamine, diisopropylethylamine (DIPEA) and mixtures thereof.

According to a particular embodiment, the catalytic hydrogenation is performed at a hydrogen pressure comprised between 0.5 and 20, preferably between 0.5 and 10, more preferably between 1 and 5, more preferably between 1 and 2 atmospheres.

Any solvent used in this type of reaction is valid. According to a particular embodiment, the solvent is a slightly polar solvent, for example, an ether, a hydrocarbon and a halogenated hydrocarbon. According to a particular embodiment, the solvent is tetrahydrofuran or dichloromethane.

According to a particular embodiment, after the catalytic hydrogenation, the process comprises adding an acid medium. According to this particular embodiment, said process comprises adding an acid which is selected from any organic or inorganic acid. Illustrative, non-limiting examples of acids suitable for this isomerization include sulfuric acid, hydrobromic acid, perchloric acid, methanesulfonic acid, hydrochloric acid or mixtures thereof. In a particular embodiment of the invention, the acid is hydrochloric acid.

Depending on the nature of the substrate and on the reaction conditions, the transformation of the compounds of formula (III) into compounds of formula (V) can take place through intermediates of formula (IV). Said compounds of formula (IV) can be isolated to later subject them to the reaction in an acid medium to obtain the compounds of formula (V).

### Preparation of the compounds of formula (III)

The compounds of formula (III) can be prepared from compounds of general formula (II). According to a particular embodiment, in the process of the invention the compound of formula (III) or a solvate thereof is obtained by subjecting a compound of formula (II) or a solvate thereof to a vinylogous Mannich reaction, 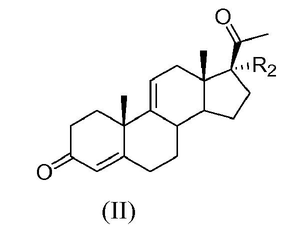 wherein R² is as defined above.

Said particular embodiment therefore comprises the regioselective olefination of position 6 of the compounds of general formula (II) by means of a vinylogous Mannich reaction. The conditions for performing said vinylogous Mannich reaction are common general knowledge, and in each case the person skilled in the art can choose the most suitable conditions (see for example conditions for the Mannich reaction described on pages 1189-1191 of March, J. "Advanced Organic Chemistry: Reactions, Mechanisms and Structure", Wiley-Interscience, fifth edition; or on pages 58-62 of Carey, F. and Sundberg, J. R. "Advanced Organic Chemistry - Part B: Reactions and Synthesis" Plenum, second edition).

The vinylogous Mannich reaction initially involves the formation of a bis-enol ether. Said bis-enol ether formation can be carried out by using a carboxylic acid orthoester, preferably a formic acid or acetic acid orthoester. In particular, ethyl and methyl orthoformate are preferred reactants for this transformation. The presence of an acid catalyst such as p-toluenesulfonic acid monohydrate, sulfosalicylic acid, perchloric acid or adipic acid or mixtures thereof allows the formation of the bis-enol ether at room temperature in a short time period, typically comprised between 15 minutes and 2 hours. P-toluenesulfonic acid monohydrate is preferably used as a catalyst. In addition, the reaction can be carried out in different organic solvents such as dioxanes, tetrahydrofuran, ethanol or mixtures thereof.

Once the bis-enol ether is formed, a compound comprising an iminium cation or the precursors of a compound comprising an iminium cation is added, a reaction occurring between position 6 of the bis-enol ether and the iminium cation. For example, *N*-methylaniline and formaldehyde can be added to introduce a methylene moiety in position 6. In a particular embodiment, the reaction with the iminium cation takes place at a temperature comprised between room temperature and the reflux temperature of the solvent used for a time period equal to or greater than 15 minutes, typically comprised between 30 minutes and 12 hours.

The formation of the exocyclic double bond in position 6 is then obtained by means of elimination in an acid medium. Illustrative, non-limiting examples of suitable acids include sulfuric acid, hydrobromic acid, perchloric acid, hydrochloric acid or any other similar inorganic acid. In a particular embodiment, the acid is hydrochloric acid.

### Preparation of the compounds of formula (I)

As has been indicated above, an additional aspect of the invention is a process for obtaining a compound of formula (I) or a solvate thereof, comprising forming a silyl enol ether derivative of a compound of formula (V) or a solvate thereof, and then reacting said resulting silyl enol ether with a quinone and a base B.

The formation of silyl enol ethers is a known process and can be performed following the conditions described in different reference books. See for example pages 793-794 of March, J. "Advanced Organic Chemistry: Reactions, Mechanisms and Structure", Wiley-Interscience, fifth edition. According to a preferred embodiment, the formation of the silyl enol ether derivative of a compound of formula (V) comprises the reaction in the presence of a base C and a silylating agent. According to a particular embodiment, said silylating agent has the formula YSi(R⁶)(R⁷)(R⁸), wherein R⁶, R⁷ and R⁸, independently of one another, represent C₁-C₆ alkyl or C₃-C₆ cycloalkyl, and Y is a leaving group, for example, halide or triflate. According to a particular embodiment, the silylating agent is a bistrimethylsilyltrihaloacetamide (wherein halo is a halogen such as fluorine, chlorine, bromine or iodine), bistrimethylsilylacetamide, hexamethyldisilazane, bistrimethylsilylurea, *tert*-butyldimethylsilyl triflate or mixtures thereof, preferably *tert*-butyldimethylsilyl triflate (TBDMSOTf).

Regardless of the method used, once obtained, said silyl enol ether is reacted with a quinone and a base B. Any reactive quinone (also known as "benzoquinones") can be used in said process, understanding "reactive quinone" as a quinone with sufficient redox potential to react with the silyl enol ether derivative of a compound of formula (V) and be subsequently eliminated, giving rise to the compounds of formula (I) or solvates thereof. A large number of quinones are known, from which the person skilled in the art can choose the most suitable one. For example, reference is made to "Comprehensive Organic Chemistry, Oxidation", Volume 7, pages 135-139. According to a particular embodiment, the quinone is a quinone of formula (Q1) or (Q2) 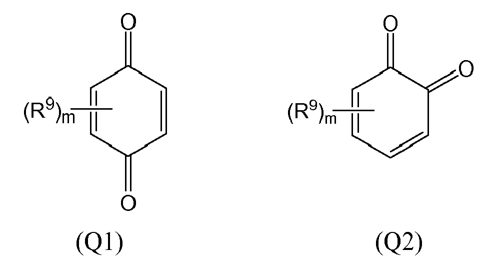 wherein each R⁹ is individually selected from the group consisting of hydrogen, C₁-C₆ alkyl, C₁-C₆ alkoxyl, halo, nitro and cyano; and m is an integer selected from 0, 1, 2, 3 and 4.

According to a particular embodiment, the quinone is a 1,4-quinone (Q1), more particularly it is selected from the group consisting of 2,3,5,6-tetrachloro-1,4-benzoquinone (*p*-chloranil) and 2,3-dichloro-5,6-dicyanobenzoquinone (DDQ) or mixtures thereof. According to a particular embodiment, the quinone is *p*-chloranil.

In another particular embodiment, in the compounds of formula (I) R² is OH or OAc. In another particular embodiment, in the compounds of formula (I) R¹ is CH₃.

The person skilled in the art can selected the most favorable reaction conditions in each case. The reaction is carried out in a suitable solvent which, according to a particular embodiment, is an ether, for example, an aliphatic ether (e.g., diisopropyl ether) or a cyclic ether (e.g., tetrahydrofuran (THF), dioxanes). According to another particular embodiment, the solvent is a halogenated solvent, for example, a chlorinated hydrocarbon (e.g., dichloromethane). According to another particular embodiment, the solvent is aromatic, for example, benzene, toluene, xylene or pyridine. According to another particular embodiment, the reaction temperature is comprised between 10°C and the reflux temperature of the solvent used. According to another particular embodiment, the reaction time is equal to or greater than 15 minutes, typically comprised between 30 minutes and 48 hours.

In another particular embodiment, the compound of formula (I) is a compound of formula (Ia) 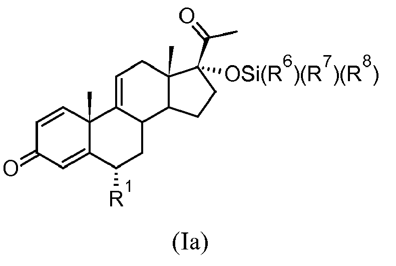 wherein R¹, R⁶, R⁷ and R⁸ are as defined above; The compound of formula (Ia) can be obtained from the compound of formula (V) as a main product or as part of a mixture together with other compounds of formula (I). Said compound of formula (Ia) can be deprotected to provide the corresponding compound of formula (I) wherein R² is hydroxyl. Said deprotection can be carried out without isolating the compound of formula (Ia) ("one-pot") or, alternatively, first isolating the compound of formula (Ia) and then subjecting it to a deprotection reaction. According to a particular embodiment, said deprotection is performed in the presence of fluoride ion (e.g., tetrabutylammonium fluoride (TBAF)). Therefore, according to a particular embodiment, the process of the invention comprises
a) forming the silyl enol ether derivative of a compound of formula (V) or a solvate thereof, wherein R² is hydroxyl;
b) reacting the intermediate formed in the presence of a quinone and a base B to provide a compound of formula (Ia) or a solvate thereof; and
c) subjecting said compound of formula (Ia) or a solvate thereof to a hydroxyl group deprotection reaction, optionally followed by an acetylation.

According to a particular embodiment, the reaction in step a) comprises adding between 1 and 5, preferably between 2 and 5 equivalents of said silylating agent for every equivalent of compound of formula (V). The amount of equivalents will depend on the conditions used and on the nature of the starting material. For example, the presence of a hydroxyl group in position 17 of the compound of formula (V) can make it necessary to add more equivalents of silylating agent. This is a situation in which the process of the invention can subsequently give rise to a compound of formula (Ia).

According to a particular embodiment, bases B and C are each independently selected from the group of trisubstituted amines, for example, a substituted amine of formula N(R₁₀)₃, wherein each R₁₀ is independently selected from a C₁-C₆ alkyl (for example trimethylamine, triethylamine, diisopropylethylamine (DIPEA) and mixtures thereof). According to another particular embodiment, said substituted amine is an aromatic amine (for example pyridine or a pyridine ring substituted with one, two or three C₁-C₆ alkyl groups).

According to a particular embodiment, said base C is selected from a trialkylamine of formula N(R₁₀)₃, wherein each R₁₀ is independently selected from a C₁-C₆ alkyl, particularly from the group consisting of trimethylamine, triethylamine, diisopropylethylamine (DIPEA) and mixtures thereof.

According to a particular embodiment, said base B is an aromatic amine, for example, pyridine or a pyridine ring substituted with one, two or three C₁-C₆ alkyl groups.

In a particular embodiment, the formation of the silyl enol ether derivative of a compound of formula (V) is carried out by using t-butyldimethylsilyl triflate (TBDMSOTf), which is then reacted in the presence of chloranil and triethylamine or pyridine.

### Transformation of the compounds of formula (I) and of formula (V) into compounds of formula (VII)

The compounds of formula (I), regardless of having been obtained from compound (V), can be transformed into fluorometholone and derivatives thereof according to processes described in the state of the art. For example, in US 2,867,637 a compound of formula (I) (R¹ = Me, R² = OH) is transformed into fluorometholone by means of a sequence involving the epoxidation of the 9-11 double bond and its subsequent opening in the presence ofHF (see Example 6 of US 2,867,637).

According to a particular embodiment, the present invention comprises the additional step of subjecting a compound of formula (I) or a solvate thereof to an epoxidation reaction to obtain a compound of formula (VI) or a solvate thereof, 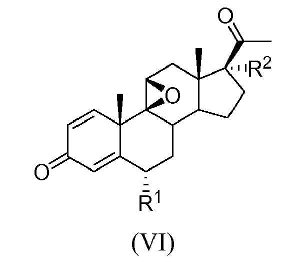 wherein R¹ and R² are as defined above.

The conditions for performing this transformation can be those described in Example 6 of US 2,867,637 or others of common general knowledge (for example, see pages 1051-1054 of March, J. "Advanced Organic Chemistry: Reactions, Mechanisms and Structure", Wiley-Interscience, fifth edition).

According to said particular embodiment, said compound of formula (VI) or a solvate thereof is reacted in the presence of a fluoride ion source to obtain a compound of formula (VII) or a solvate thereof, 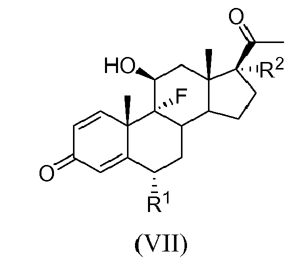 wherein R¹ and R² are as defined above.

The conditions for performing this transformation can be those described in Example 6 of US 2,867,637 or others of common general knowledge (for example, see pages 520 and 521 of March, J. "Advanced Organic Chemistry: Reactions, Mechanisms and Structure", Wiley-Interscience, fifth edition).

According to said particular embodiment, said compound of formula (VII) is fluorometholone (compound of formula (VII) wherein R¹ is CH₃ and R² is -OH) or the acetate thereof in position 17 (compound of formula (VII) wherein R¹ is methyl and R² is -O(C=O)CH₃).

### Other transformations

The process of the present invention also includes additional transformations of functional groups in the described compounds. For example, it is possible to transform a compound of given general formula wherein R² is hydroxyl into another compound of the same formula in which R² is -O(C=O)CH₃ by means of transformations known by the person skilled in the art.

According to a particular embodiment, the compounds of formula (I) can be transformed into other different compounds of formula (I), which transformations are also included in the scope of the present invention. For example, several transformations of the R² group can be performed, including, among others, protection and/or deprotection, alkylation or acetylation, according to known methods.

According to additional particular embodiments, said reactions can be performed on compounds of formula (I), (Ia), (II), (III), (IV), (V), (VI) or (VII).

For example, in the event that R² is -OC(=O)R⁴, a compound of formula (I), (II), (III), (IV), (V), (VI) or (VII) can be subjected to a hydrolysis reaction to give rise to the corresponding hydroxyl group, according to methods known by the person skilled in the art (for example, see hydrolysis conditions described on pages 469-474 of March, J. "Advanced Organic Chemistry: Reactions, Mechanisms and Structure", Wiley-Interscience, fifth edition). In a particular embodiment, the hydrolysis carried out in a basic medium. According to a particular embodiment, hydrolysis is performed in the presence of KOH, NaOH, MeOH/H₂O or NaOH/TBAH in THF.

The opposite reaction is also possible, i.e., in the event that R² is a hydroxyl, a compound of formula (I), (II), (III), (IV), (V), (VI) or (VII) can be transformed into a OC(=O)R⁴ group, for example into an acyl group, according to methods known by the person skilled in the art (for example, see esterification conditions described on pages 484-486 of March, J. "Advanced Organic Chemistry: Reactions, Mechanisms and Structure", Wiley-Interscience, fifth edition). In a particular embodiment, the esterification is carried out in a basic medium. According to a particular embodiment, the acetylation is performed in the presence of Ac₂O/TsOH in AcOH.

These additional transformations of functional groups can also serve to prepare the starting compounds. The compounds of formula (II) in which R₂ is -OH can be obtained commercially. Said hydroxyl group can be acetylated or protected by the methods described in the present application to provide other compounds of formula (II) in which R₂ is different from -OH.

### Intermediates of the process

Additional aspects of the invention are the compounds of formula (Ia), (III) and (IV) or solvates thereof, defined above, novel intermediates in the preparation of the compounds of formula (I) and of formula (V). The processes for obtaining them are also aspects of the present invention.

An additional aspect of the present invention is a process for synthesizing a compound of formula (Ia) or a solvate thereof, as has been defined above, comprising forming the silyl enol ether derivative of a compound of formula (V) or a solvate thereof, 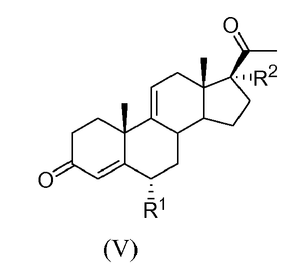 wherein R¹ is as defined above, and R₂ is hydroxyl;
and reacting said resulting silyl enol ether with a quinone and a base B.

An additional aspect of the present invention is a process for synthesizing a compound of formula (IV) or a solvate thereof, as defined above, comprising the catalytic hydrogenation of a compound of formula (III) or a solvate thereof, 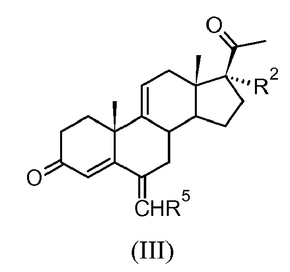 wherein R⁵ and R² are as defined above.

An additional aspect of the present invention is a process for obtaining a compound of formula (III) or a solvate thereof, as defined above, comprising subjecting to a vinylogous Mannich reaction a compound of formula (II) or a solvate thereof, 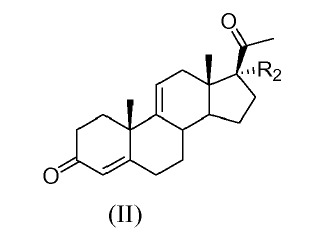 wherein R² is as defined above.

### EXAMPLES

### Example 1: Preparation of 17α-Hydroxy-6-methylene-4,9(11)-pregnadiene-3,20-dione (compound of formula III)

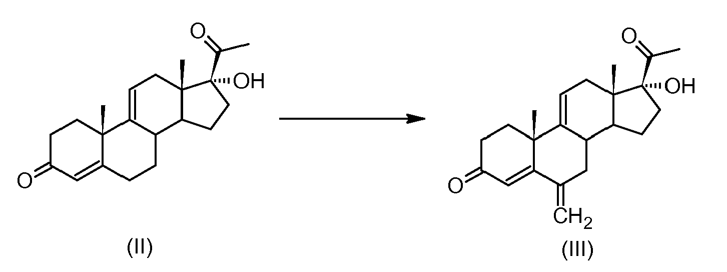

1.94 g of 17α-hydroxy-Δ⁹⁽¹¹⁾-progesterone (II) (5.91 mmol) are suspended in 13.5 mL of THF, 7.74 g of triethyl orthoformate (7.98 mmol) and 1.6 mL of ethanol are added. The temperature is adjusted to 37 ± 5°C and 0.05 g of p-toluenesulfonic acid monohydrate (0.24 mmol) are added. The stirring is maintained for two hours and then 0.66 g of *N*-methylaniline (6.15 mmol) and 0.55 mL of formaldehyde (35%) are added and the temperature is adjusted to 42 ± 5°C. After stirring for one and a half hours, it is cooled to 22 ± 5°C and 3.9 mL of commercial hydrochloric acid are added without the temperature exceeding 40°C. It is stirred for two hours. Then, 13.5 mL of water are added and it is cooled to 5 ± 5°C. It is stirred for 30 minutes, filtered and washed with water. The solid is resuspended in water at 15 ± 5°C, filtered, washed with cold water and dried.
Molar yield: 78.2%
¹H-NMR (CDCl₃, 200 MHz): δ= 0.7 (3H, s, C19), 1.2 (3H, s, C18), 0.5-3.0 (15H, m), 2.3 (3H, s, C21), 5.0 (1H, s, C22), 5.1 (1H, s, C22), 5.6 (1H, d, C11), 5.9 (1H, s, C4).
¹³C-NMR (CDCl₃) 50 MHz: δ= 15.22 (q, C19), 24.58 (t, C15), 26.20 (q, C21), 27.48 (q, C18), 32.25 (t, C16), 33.27 (t, C7), 33.74 (t, C12), 34.01 (t, C2), 37.78 (d, C8), 40.01 (t, C1), 41.38 (s, C10), 46.77 (s, C13), 47.98 (d, C14), 89.46 (s, C17), 114.00 (t, C22), 119.18 (d, C11), 121.68 (d, C4), 143.11 (s, C9), 145.47 (s, C6), 167.42 (s, C5), 199.50 (s, C3), 211.13 (s, C20).
HRMS-EI(M+Na⁺): Calculated for C₂₂H₂₉O₃: 341.2111, experimental: 341.2112.

### Example 2: Preparation of 17α-Acetoxy-Δ⁹⁽¹¹⁾-progesterone (compound of formula II)

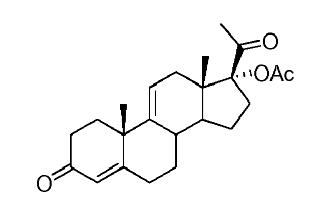

11 g of 17α-hydroxy-Δ⁹⁽¹¹⁾-progesterone (compound of formula II wherein R² is hydroxyl) (33.5 mmol) are suspended in dichloromethane. Acetic anhydride (201 mmol) and perchloric acid (70%) (83.7 mmol) at -5°C are added on this mixture. The stirring is maintained at this temperature for 22 hours and the obtained product is purified by means of column chromatography.
¹H-NMR (CDCl₃, 200 MHz): δ= 0.5 (3H, s, C19), 1.3 (3H, s, C18), 0.5-3.0 (16H, m), 2.0 (3H, s, C21), 2.0 (3H, s, OAc), 5.5 (1H, d, C11), 5.7 (1H, s, C4).
¹³C-NMR (CDCl₃, 50 MHz): δ= 14.21 (q, C19), 20.99 (q, OAc), 24.52 (t, C16), 26.13 (q, C21), 26.13 (q, C18), 30.11 (t, C15), 32.05 (t, C7), 32.64 (t, C6), 32.81 (t, C2), 33.76 (t, C1), 34.13 (t, C12), 37.40 (d, C8), 40.88 (s, C10), 45.20 (s, C13), 48.24 (d, C14), 96.27 (s, C17), 118.23 (d, C11), 123.92 (d, C4), 144.19 (s, C9), 168.98 (s, C5), 170.31 (s, OAc), 198.62 (s, C3), 203.47 (s, C20).
HRMS-EI(M+Na⁺): Calculated for C₂₃H₃₀O₄Na: 393.2036, experimental: 393.2046.

### Example 3: 17α-Acetoxy-6-methylene-4,9(11)-pregnadiene-3,20-dione (compound of formula III)

17α-Acetoxy-6-methylene-4,9(11)-pregnadiene-3,20-dione was prepared from the compound obtained in Example 2 following conditions similar to those of Example 1. 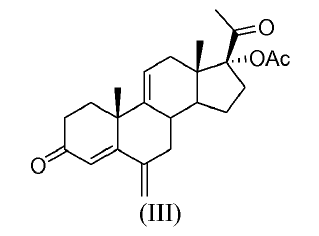 ¹H-NMR (CDCl₃, 200 MHz): δ= 0.6 (3H, s, C19), 1.2 (3H, s, C18), 0.5-3.1 (14H, m), 2.0 (3H, s, C21), 2.1 (3H, s, OAc), 5.0 (1H, s, C22), 5.1 (1H, s, C22), 5.6 (1H, s, C11), 5.9 (1H, s, C4).
¹³C-NMR (CDCl₃, 50 MHz): δ= 14.34 (q, C19), 21.14 (q, OAc), 24.53 (t, C16), 26.20 (q, C21), 26.33 (q, C18), 30.26 (t, C15), 32.93 (t, C7), 33.29 (t, C2), 34.02 (t, C1), 37.71 (d, C8), 39.86 (t, C12), 41.36 (s, C10), 45.40 (s, C13), 48.76 (d, C14), 96.26 (s, C17), 114.20 (d, C22), 118.82 (d, C11), 121.79 (d, C4), 143.25 (s, C6), 145.20 (s, C9), 167.02 (s, C5), 170.46 (s, OAc), 199.25(s, C3), 203.69 (s, C20).

### Example 4: Preparation of 17α-Hydroxy-6α-methyl-4,9(11)-pregnadiene-3,20-dione (compound of formula V)

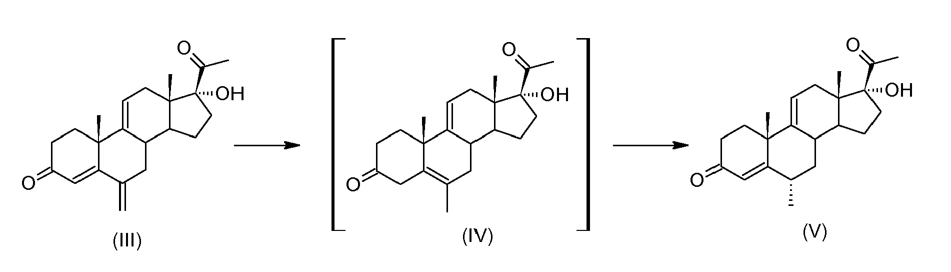

18.40 g of 17α-hydroxy-6-methylene-4,9(11)-pregnadiene-3,20-dione (III) (54.10 mmol) are suspended in 350 mL of dichloromethane and 1.84 g of 10% Pd/C (50% moist) and 5.25 mL of triethylamine (37.87 mmol) are added at room temperature. The reaction is maintained under an atmosphere of H₂ at a pressure of 1.1 atmospheres and it is stirred for six hours to give 17α-hydroxy-6-methyl-5,9(11)-pregnadiene-3,20-dione (IV) which is not isolated. The reaction mixture is diluted in 350 mL of THF, 11.27 mL of concentrated HCl (135.25 mmol) are slowly added and the stirring is maintained at room temperature for 14 hours. Finally, the solvents are eliminated by means of reduced-pressure distillation and 200 mL of MeOH are added, the resulting solution is slowly poured on 200 mL of water at 0°C, filtered, washed with water at 0°C and dried.
Molar yield: 85.5%
¹H-NMR (CDCl₃, 400 MHz): δ= 0.7 (3H, s, C19), 1.1 (3H, d, C22), 1.3 (3H, s, C18), 2.3 (3H, s, C21), 0.5-3.0 (16H, m), 5.5 (1H, d, C11), 5.8 (1H, s, C4).
¹³C-NMR (CDCl₃, 100 MHz): δ= 15.26 (q, C19), 18.11 (q, C22), 24.60 (t, C15), 27.20 (q, C18), 27.51 (q, C21), 32.15 (t, C16), 33.67 (t, C2), 33.80 (d, C6), 33.98 (t, C1), 33.98 (t, C12), 37.23 (d, C8), 41.34 (t, C7), 46.63 (s, C13), 47.35 (d, C14), 89.47 (s, C17), 118.35 (d, C11), 121.16 (d, C4), 143.95 (s, C9), 172.77 (s, C5), 199.62 (s, C3), 211.24 (s, C20).
HRMS-EI(M+Na⁺): Calculated for C₂₂H₃₀O₃Na: 365.2087, experimental: 365.2098.

### Example 5: Preparation of 17α-hydroxy-6-methyl-5,9(11)-pregnadiene-3,20-dione (compound of formula IV)

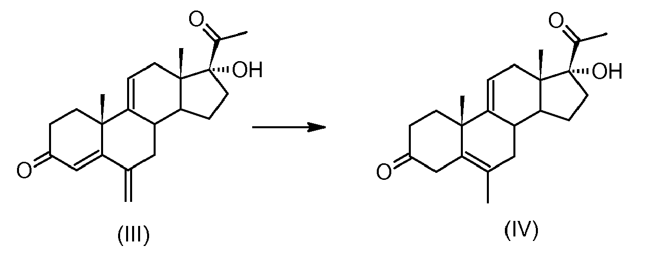

18.40 g of 17α-hydroxy-6-methylene-4,9(11)-pregnadiene-3,20-dione (III) (54.10 mmol) are suspended in 350 mL of ethyl acetate and 1.84 g of 10% Pd/C (50% moist) and 5.25 mL of triethylamine (37.87 mmol) are added at room temperature. The reaction is maintained under an atmosphere of H₂ at a pressure of 1.1 atmospheres and it is stirred for six hours. It is filtered through Celite, concentrated to a volume of 37 mL and 55 mL of heptane are loaded. It is filtered at 20/25° C and washed with heptane. Molar yield: 55.5%
¹H-NMR (CDCl₃, 400 MHz): δ= 0.6 (3H, s), 1.2 (3H, s), 1.4 (1H, m), 1.6 (3H, s), 1.6-2.2 (9H, m), 2.2 (3H, s), 2.3-2.7 (4H, m), 3.0-3.3 (2H, m), 5.5 (1H, d) ppm.
¹³C-NMR (CDCl₃, 400 MHz): δ= 15.19 (CH₃), 19.42 (CH₃), 24.90 (CH₂), 27.73 (CH₃), 32.28 (CH₂), 33.96 (CH₂), 34.69 (CH), 35.06 (CH₂), 37.63 (CH₂), 38.50 (C), 39.11 (C), 39.32(CH₂), 42.79 (CH₂), 48.18 (CH), 89.92 (C), 117.25 (CH), 127.20 (C), 129.02 (C), 144.54 (C), 210.91 (C), 211.72 (C) ppm.

### Example 6: Preparation of 17-Hydroxy-6α-methyl-4,9(11)-pregnadiene-3,20-dione (compound of formula V)

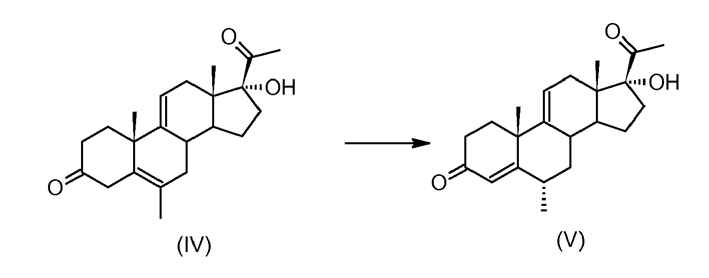

13.7 g of 17α-hydroxy-6-methyl-5,9(11)-pregnadiene-3,20-dione (IV) (40 mmol) are dissolved in 350 mL of THF and 350 mL of DCM, 11.27 mL of concentrated HCl (135.25 mmol) are slowly added and the stirring is maintained at room temperature for 14 hours. Finally, the solvents are eliminated by means of reduced-pressure distillation and 200 mL of MeOH are added, the resulting solution is slowly added on 200 mL of water at 0°C, filtered, washed with water at 0°C and dried.
Molar yield: 90%

### Example 7: Preparation of 17α-Hydroxy-6α-methyl-1,4,9(11)-pregnatriene-3,20-dione (compound of formula I)

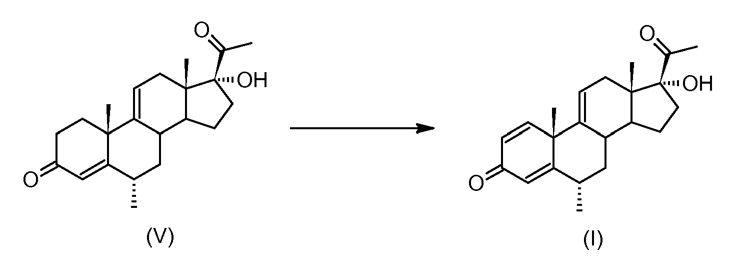

3.67 g of 17α-hydroxy-6α-methyl-4,9(11)-pregnadiene-3,20-dione (V) (10.73 mmol) are suspended in 37.6 mL of dichloromethane under an inert atmosphere and the temperature is adjusted to 10°C. 6.1 mL of DIPEA (diisopropylethylamine) (34.91 mmol) and 6.3 mL of TfO-TBDMS (t-butyldimethylsilyl triflate) (27.27 mmol) are added. The stirring is maintained for 5 hours and 60.2 mL of water are added and it is allowed to react for 45 minutes and the phases are separated. The temperature of the organic phase is adjusted to 15°C and 1.8 mL of pyridine (21.82 mmol) and 2.95 g of chloranil (12.00 mmol) are added. It is stirred for 14 hours and the resulting reaction mixture is filtered on Celite, washed with dichloromethane and the organic phase is separated. The residue thus obtained is redissolved in 37.6 mL of THF and allowed to react with 10.32 g of tetrabutylammonium fluoride (32.73 mmol) for 2.5 days. Finally, water is added and it is extracted with AcOEt. The solvent is evaporated and it is redissolved in 10 mL of MeOH and poured on water at 0°C. The obtained solid is filtered and washed with water at 0°C.
Molar yield: 80%
¹H-NMR (CD₃Cl) 200 MHz: δ= 0.7 (3H, s, C19), 1.1 (3H, d, C22), 1.4 (3H, s, C18), 2.3 (3H, s, C21), 0.5-3.0 (12H, m), 5.5 (1H, d, C11), 6.1 (1H, s, C4), 6.3 (1H, d, C1), 7.2 (1H, d, C2).
¹³C-NMR (CD₃Cl) 50 MHz: δ= 15.15 (q, C19), 17.57 (q, C22), 24.84 (t, C15), 27.06 (q, C18), 27.48 (q, C21), 32.32 (t, C16), 33.21 (d, C6), 33.74 (t, C7), 36.57 (d, C8), 43.58 (t, C12), 46.06 (s, C10), 46.87 (s, C13), 47.75 (d, C14), 89.40 (s, C17), 120.35 (d, C11), 121.06 (d, C4), 126.93 (d, C1), 142.71 (s, C9), 155.16 (d, C2), 169.72 (s, C5), 186.43 (s, C3), 211.02 (s, C20).
HRMS-EI(M+Na⁺): Calculated for C₂₂H₂₈O₃Na: 363.1931, experimental: 363.1939.

### Example 8: Preparation of 17α-Tert-butyldimethylsilyloxy-6α-methyl-1,4,9(11)-pregnatriene-3,20-dione (compound of formula Ia)

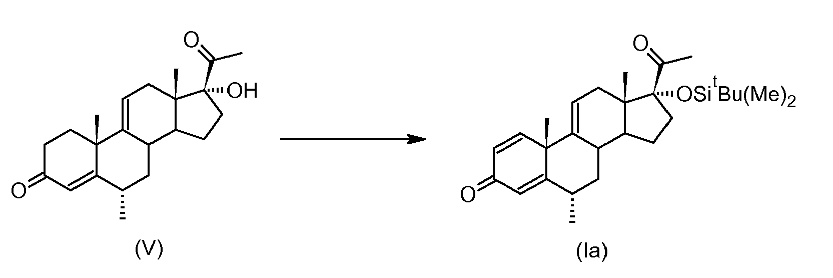

5.50 g of 17α-hydroxy-6α-methyl-4,9(11)-pregnadiene-3,20-dione (V) (16.09 mmol) are suspended in 37.6 mL of dichloromethane under an inert atmosphere and the temperature is adjusted to 10°C. 9.15 mL of DIPEA (diisopropylethylamine) (52.4 mmol) and 9.45 mL of TfO-TBDMS (t-butyldimethylsilyl triflate) (40.91 mmol) are added. The stirring is maintained for 5 hours and 60.2 mL of water are added and it is allowed to react for 45 minutes. The temperature of the organic phase is adjusted to 15°C and 1.8 mL of pyridine (21.82 mmol) and 2.95 g of chloranil (12.00 mmol) are added. It is stirred for 14 hours and the resulting reaction mixture is filtered on Celite and washed with dichloromethane. The phases are separated and the organic phase is evaporated. The obtained crude reaction product is purified by means of column chromatography.
Molar yield: 80%
¹H-NMR (CDCl₃, 200 MHz): δ= 0.0 (3H, s, Si-Me), 0.1 (3H, s, Si-Me), 0.6 (3H, s, C19), 0.9 (9H, s, Si-tBu), 1.2 (3H, d, C22), 1.4 (3H, s, C18), 0.6-3.0 (14H, m), 5.5 (1H, s, C11), 6.1 (1H, s, C4), 6.3 (1H, d, C1), 7.2 (1H, s, C2).

### Example 9: Preparation of 17α-Hydroxy-6α-methyl-9(11)β-epoxy-1,4-pregnadiene-3,20-dione (compound of formula VI)

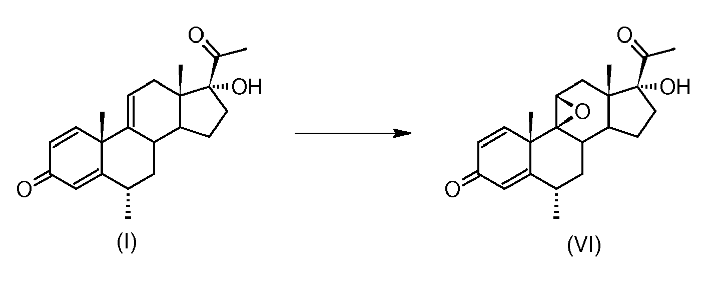

1.98 g of 17α-hydroxy-6α-methyl-1,4,9(11)-pregnatriene-3,20-dione (I) (5.82 mmol) are suspended in a mixture of 20 mL of acetone:water in a 5.3:1 ratio. 0.07 mL of 70% HClO₄ (1.16 mmol) are added and the temperature is adjusted to 25°C. 1.17 g of DDH (dibromodimethylhydantoin) (4.07 mmol) are added. It is allowed to react under stirring for 4 and a half hours and the reaction mixture is poured on a solution of 3.62 g of K₂CO₃ (26.19 mmol) and 5 mL of 5% NaHSO₃ in a mixture of 40 mL of acetone:water in a 1:1 ratio and it is allowed to react for 4 hours. Finally, glacial is added to pH=7, and the acetone is distilled. It is cooled to 0°C and the obtained solid is filtered, washed with water at 0°C, dried and weighed (Yield 80%).
¹H-NMR (CDCl₃, 200 MHz): δ= 0.8 (3H, s, C19), 1.1 (3H, d, C22), 1.4 (3H, s, C18), 2.2 (3H, s, C21), 0.5-3.5 (13H, m), 6.1 (1H, s, C4), 6.2 (1H, d, C1), 6.5 (1H, d, C2).
¹³C-NMR (CDCl₃, 50 MHz): δ= 17.43 (q, C19), 17.94 (q, C22), 24.07 (q, C18), 24.80 (t, C15), 27.31 (q, C21), 30.63 (t, C16), 31.60 (d, C6), 32.75 (t, C7), 34.39 (d, C8), 42.35 (t, C12), 44.25 (s, C10), 46.25 (s, C13), 50.17 (d, C14), 63.44 (d, C11), 67.02 (s, C9), 89.18 (s, C17), 121.58 (d, C4), 127.61 (d, C1), 152.86 (d, C2), 168.36 (s, C5), 186.28 (s, C3), 210.34 (s, C20).
HRMS-EI (M+Na⁺): Calculated for C₂₂H₂₈O₄Na: 379.1879, experimental: 379.1870.

### Example 10: Preparation of Fluorometholone (compound of formula VII)

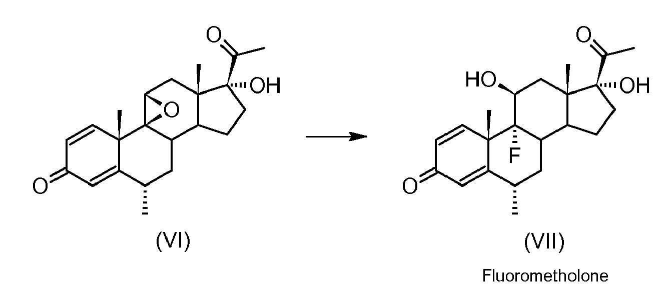

1 g of 17α-hydroxy-6α-methyl-9(11)β-epoxy-1,4-pregnadiene-3,20-dione (VI) is added on 2 mL of HF (70%) at -50°C, stirred until the end of the reaction and poured on 50 mL of an aqueous solution of ammonium (20%) at 0-5°C. It is filtered, washed with water and dried.

The resulting compound coincides with the fluorometholone described in the Pharmacopoeia.

## Claims

1. A process for obtaining a compound of formula (V) or a solvate thereof, 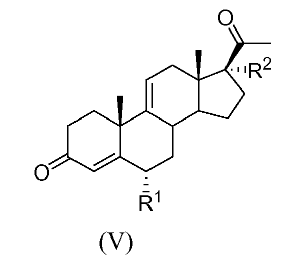 wherein
R¹ is C₁-C₆ alkyl;
R² is -OR³, -OC(=O)R⁴ or -O-(HPG), wherein
R³ is H, C₁-C₆ alkyl or C₆-C₁₄ aryl;
R⁴ is H or C₁-C₆ alkyl; and
HPG is a hydroxyl protecting group;
comprising the catalytic hydrogenation of a compound of formula (III) or a solvate thereof, 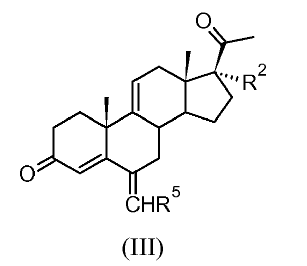 wherein R² is as defined above, and R⁵ is hydrogen or C₁-C₅ alkyl.

2. The process according to claim 1
comprising the catalytic hydrogenation in the presence of a base A of a compound of formula (III) or a solvate thereof, 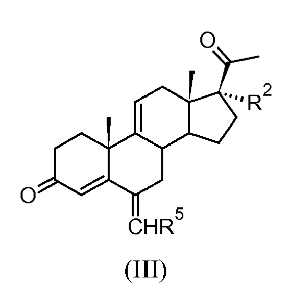 wherein R² is as defined above, and R⁵ is hydrogen or C₁-C₅ alkyl;
followed by reaction in an acid medium.

3. The process according to claim 1 or 2, wherein R¹ is methyl and/or R² is hydroxyl or acetate.

4. The process according to any of the previous claims, comprising forming a silyl enol ether derivative of the compound of formula (V) or a solvate thereof;
and then the reaction with a quinone and a base B to obtain a compound of formula (I) or a solvate thereof 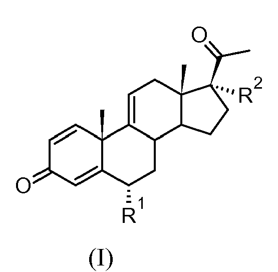 wherein R¹ and R² are as defined in claim 1.

5. The process according to claim 4, wherein
a) said compound of formula (I) or a solvate thereof is subjected to an epoxidation reaction to obtain a compound of formula (VI) or a solvate thereof, 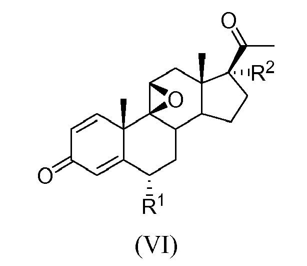 wherein R¹ and R² are as defined in claim 1; and
b) said compound of formula (VI) or a solvate thereof is reacted in the presence of a fluoride ion source to obtain a compound of formula (VII) or a solvate thereof, 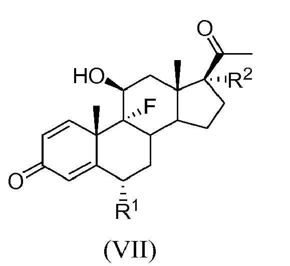 wherein R¹ and R² are as defined in claim 1.

6. A process for obtaining a compound of formula (V) or a solvate thereof, 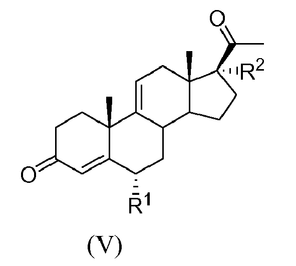 wherein
R¹ is C₁-C₆ alkyl;
R² is -OR³, -OC(=O)R⁴ or -O-(HPG), wherein
R³ is H, C₁-C₆ alkyl or C₆-C₁₄ aryl; R⁴ is H or C₁-C₆ alkyl; and HPG is a hydroxyl protecting group;
comprising reacting in an acid medium a compound of formula (IV) or a solvate thereof, 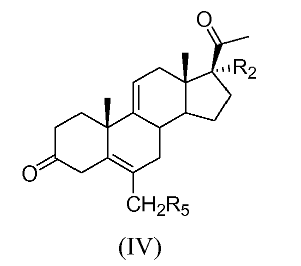 wherein R⁵ is hydrogen or C₁-C₅ alkyl; and R² is as defined above.

7. A compound of formula (IV) or a solvate thereof 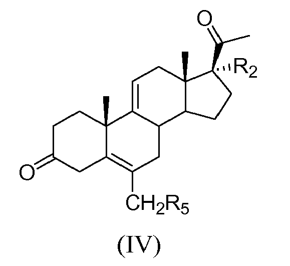 wherein R⁵ and R² are as defined in claim 1.

8. A compound of formula (III) as defined in claim 1 or a solvate thereof 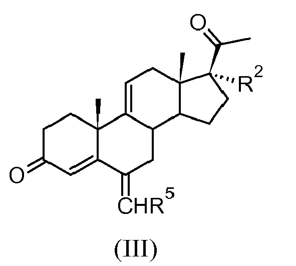 5 wherein R⁵ and R² are as defined in claim 1.

9. A process for obtaining a compound of formula (IV) or a solvate thereof as defined in claim 7, comprising the catalytic hydrogenation of a compound of formula (III) or a solvate thereof, 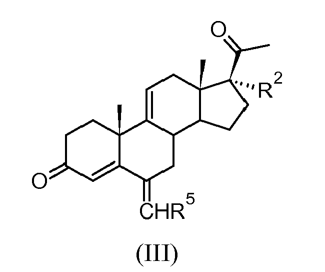 wherein R⁵ and R² are as defined in claim 1.

10. A process for obtaining a compound of formula (III) or a solvate thereof as defined in claim 8, comprising subjecting a compound of formula (II) or a solvate thereof to a vinylogous Mannich reaction, 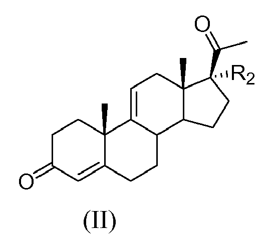 wherein R² is as defined in claim 1.

11. A process for obtaining a compound of formula (I) or a solvate thereof, 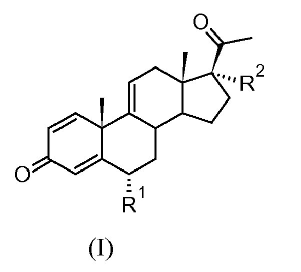 wherein
R¹ is C₁-C₆ alkyl;
R² is OR³, OC(=O)R⁴ or O-(HPG), wherein
R³ is H, C₁-C₆ alkyl or C₆-C₁₄ aryl; R⁴ is H or C₁-C₆ alkyl; and HPG is a hydroxyl protecting group;
comprising
forming a silyl enol ether derivative of a compound of formula (V) or a solvate thereof, 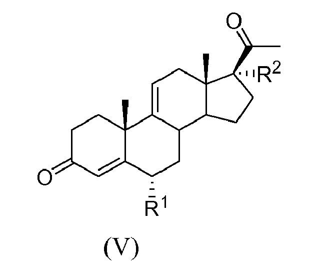 wherein R¹ and R² are as defined above;
and reacting the resulting compound with a quinone and a base B.

12. The process according to claim 11, comprising forming a silyl enol ether derivative of a compound of formula (V) or a solvate thereof, 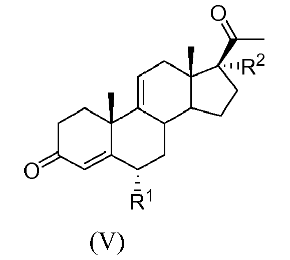 wherein R¹ is as defined in claim 11, and R₂ is a hydroxyl;
in the presence of a base C and a silylating agent of formula YSi(R⁶)(R⁷)(R⁸), wherein Y is a leaving group, and R⁶, R⁷ and R⁸, independently of one another, represent C₁-C₆ alkyl or C₃-C₆ cycloalkyl;
and reacting the resulting compound with a quinone and a base B to provide a compound of formula (Ia) or a solvate thereof, 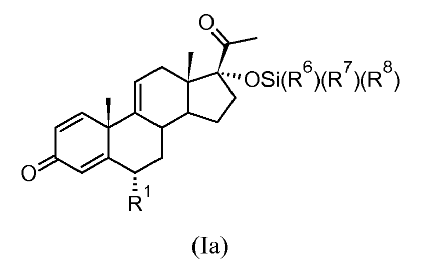 wherein R¹, R⁶, R⁷ and R⁸ are as defined above; and subjecting said compound of formula (Ia) or a solvate thereof to a hydroxyl group deprotection reaction, optionally followed by an acetylation.

13. The process according to any of claims 11 or 12, wherein
a) said compound of formula (I) or a solvate thereof is subjected to an epoxidation reaction to obtain a compound of formula (VI) or a solvate thereof, 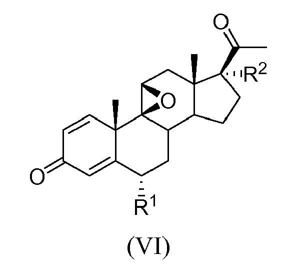 wherein R¹ and R² are as defined in claim 11; and
b) said compound of formula (VI) or a solvate thereof is reacted in the presence of a fluoride ion source to obtain a compound of formula (VII) or a solvate thereof, 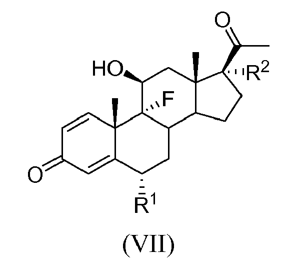 wherein R¹ and R² are as defined in claim 11.

14. A compound of formula (Ia) or a solvate thereof 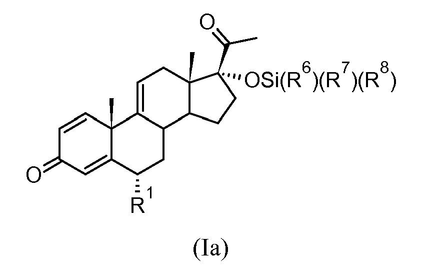 wherein
R¹ is C₁-C₆ alkyl; and
R⁶, R⁷ and R⁸, independently of one another, represent C₁-C₆ alkyl or C₃-C₆ cycloalkyl.
